# EUROPEAN PATENT APPLICATION

(11) **EP 1 870 090 A2**
(43) Date of publication of application: **26.12.2007**
(21) Application number: 06743402.7
(22) Date of filing: 13.03.2006
(51) Int. Cl.: A61K 9/12

(54) **NOVEL STABLE FORMULATION OF SUSPENDED AEROSOLS AND PRODUCTION METHOD THEREOF**

(30) Priority: 15.03.2005 ES 200500591
(71) Applicant: Laboratorio Aldo-Union, S.A., 08950 Esplugues de Llobregat (ES)
(72) Inventor: CALZADA PRATMARSÓ, Alejandra, 08950 Esplugues de Llobregat (Barcelona) (ES)
(74) Representative: SUGRANES - VERDONCES - FERREGÜELA
(86) International application number: PCT/ES2006/000120
(87) International publication number: WO 2006/097556

(57) **Abstract**

The invention relates to a stable formulation of suspended aerosols, which is intended for the inhalatory administration of medicaments, comprising one or more active principles, e.g. of the anti-inflammatory corticosteroid and bronchodilator type, less than 0.3 % p/p ethanol which acts as a dispersion adjuvant, a dispersing agent such as oleoic acid and a hydrofluorocarbonated propellant, preferably 1, 1, 1,2-tetrafluorethane (134a). According to the invention, once the ethanol has been added, the active principle and the propellant are mixed and the formulation thus prepared is dosed in aerosol containers such as to enable the correct oral inhalatory administration thereof.

## Description

### OBJECT OF THE INVENTION

The present invention relates to a novel stable formulation of suspended aerosols, based on the use of a hydrofluorocarbon as propellant, and wherein stability is achieved by adding ethanol in very small quantities and the use of valves provided with seals compatible with the formulation.

The invention also relates to the procedure used to produce these novel stable compositions.

The field of application of the present invention is centred on the pharmaceutical industry, and more specifically on the sector thereof devoted to producing aerosols.

### BACKGROUND OF THE INVENTION

In the treatment of obstructive airways diseases and asthma, the most widely used therapy on global level is constituted by pressurized inhalers which dispense exact quantities of a medicament on actuating the inhaler.

Compared with oral administration, inhalation provides a quicker start of action whilst minimizing the systemic secondary effects.

Aerosol formulations can be administered by oral inhalatory route (through the mouth) or by nasal route (by application on the nasal mucosa).

In general terms, all aerosols are formed by mechanical elements and by the formulation or set of substances it contains. The mechanical elements are the container, the valve and the button.

The formulation is the set of substances introduced inside the aerosol and comprises the active ingredient which justifies the product, auxiliary substances which facilitate the system (solvents, dispersing agents, etc) and propellants or substances which provide an internal overpressure due to their high vapour pressure.

The propellants maintain a pressure inside the container above atmospheric pressure and they are substances which have a high vapour pressure at ambient temperature (liquefied gases). The propellant provides the internal overpressure capable of pushing the content outside when the valve is actuated; without propellant the aerosol system cannot function.

The aerosol formulation can contain the medicament in solution or suspension. In the solutions, the active ingredient and the other excipients are totally dissolved and in the case of suspensions the medicament is found as a powder finely divided in excipients.

Both in solutions and in suspensions, containers are filled capable of supporting the pressure required to maintain the propellant as a liquid.

Traditionally, metered-dose inhalers have been formulated with chlorofluorocarbonated (CFC) propellants, such as freon 11 (trichlorofluoromethane), freon 12 (dichlorodifluoromethane) and freon 114 (1,2-dichloro-1,1,2,2-tetrafluoroethane). However, it has been demonstrated that these gases damage the ozone layer due to the fact that they contain chlorine atoms which are released due to their exposure to ultraviolet rays, contributing to the destruction of the ozone layers in the upper atmosphere. A committee on the ozone layers organized the preparation of the Montreal Protocol. This protocol establishes the progressive suspension of the production and consumption of substances which damage the ozone layer. Propellants alternative to CFC were researched and hydrofluoroalkanes (HFA) among which are found 1,1,1,2-tetrafluoroethane (HFA 134a) and 1,1,1,2,3,3,3-heptafluoroethane (HFA 227) were identified. Neither propellant contains chlorine and it is considered that they do not have the potential for destruction of the ozone layer.

Many of the aerosol formulations use said hydrofluorocarbonated propellants. Nevertheless, problems arise in producing preparations which are pharmaceutically acceptable due to the fact that the hydrofluorocarbonated propellants do not function well with the components that traditionally formed a formulation with CFCs.

The new propellants require changes in the additives or coadjuvants necessary so that the preparation provides a medicament with pharmaceutically suitable characteristics.

### DESCRIPTION OF THE INVENTION

Stable formulations of suspended aerosols that the invention provides resolves, in a fully satisfactory manner, the aforementioned problem in the different mentioned aspects.

The present invention relates to a stable formulation of suspended aerosol, which contains one or more active ingredients together with the suitable excipients; a composition which is packaged in aluminium bottles provided with dispensing valves which have seals compatible with said formulation.

To achieve the stability of the medicament of the invention, two critical factors are taken into consideration, which are, on the one hand, the use of ethanol in quantities below 0.30% w/w and, on the other hand, that the materials of the seals of the dispensing valves are compatible with the formulation, not observing extraction products of the valve throughout the aging of the product.

Conventionally, ethanol has been used and is currently used in numerous aerosols with hydrofluorocarbonated propellants as cosolvent or solvent to solubilize the dispersing agents, in the case of suspensions since they are not soluble in the new propellants, or to dissolve the active ingredient in the case of solutions.

The formulations recommended are characterized in that ethanol is used in very small quantities, less than 0.30 % w/w, so that its function is not that of acting as cosolvent or solvent, but as dispersion coadjuvant. The quantity of ethanol has to be the minimum so that there is no possibility that part of the active ingredient solublizes, producing a chemical degradation and increase in size of particles and making the suspension unstable.

The stable formulation of suspended aerosol that the invention proposes, comprises a medicament, ethanol, which acts as dispersion coadjuvant, oleic acid as dispersing agent and a 1,1,1,2-tetrafluoroethane (HFA-134a) as propellant.

The active ingredients used in the present invention are selected from any suitable substance to be administered by a metered-dose inhaler, and may be one or several, and in a quantity which is that necessary and effective to produce the desired therapeutic effect. They may be as free base or as a pharmaceutically acceptable salt and in no case toxic. The selection of a particular salt will depend on the nature and chemical stability of a base, and on the solubility of the salt in the formulation.

Said medicament is not soluble in the combination HFA/ ethanol, since if this was not the case, crystallization problems would arise in the produce and increase the aerosol particle size.

Among other medicaments are found the bronchodilators, in particular anticholinergics and sympathomimetics, such as for example, ipatropium, triotropium, salbutamol, salmeterol, formoterol, terbutalin, isoproterenol, reproterol, bambuterol and pirputerol and anti-inflammatory corticosteroids, including beclomethasone, budesonide, flunisolide, fluticasone and triamcinolone. Other medicaments which may be used in this type of compositions are cromoglycate and nedocromil.

With regard to the salts that may be used with the aforementioned medicaments we find ipatropium bromide, tiotropium bromide, salbutamol sulphate, salmeterol xinafoate, formoterol fumarate, terbutalin sulphate, isoproterenol sulphate, beclomethasone dipropionate, fluticasone propionate and triamcinolone acetonide.

As propellant, any of the conventional hydrofluorocarbonated propellants can be used. In a particular embodiment of this invention the hydrofluorocarbonated propellant is selected from the group formed by 1,1,1,2-tetrafluoroethane (134a), 1,1,1,2,3,3,3-heptafluoroethane (277) and their mixtures. Preferably, the propellant used is 134a.

Another of the components used in the present invention is the dispersing agent or surfactant, which acts by lubricating the valve to avoid its blocking and thus dispense exactly the same dose in each press. The dispersing agents preferably used are sorbitan trioleate and oleic acid and, especially the latter in a proportion lower than 0.050 % w/w, for example 0.020 % w/w. optionally, other dispersing agents can be added such as propylene glycol.

As has been previously commented, ethanol is found in a quantity lower than 0.30 % w/w; said choice has been made based on the results of the measurement parameters of the average aerodynamic diameter of the MMAD aerosol particles, and in the percentage of particles lower than 5 µ (FPF < 5 µ). Both parameters are obtained by cascade impacters, such as, for example, the Andersen cascade impacter. These parameters are very important for the "in vitro" characterization of an aerosol, since they will give an idea of the "in vivo" behaviour of the aerosol and its bioavailability.

According to the aforementioned, with the use of 0.25 % w/w of ethanol, the MMAD and FPF results < 5 µ, are within the determined limits of 3.20 to 3.50 µ for MMAD and from 40-60% for FPF < 5 µ, on increasing or decreasing the quantity of ethanol, the values of these two parameters would vary drastically.

The invention also relates to the method of industrial manufacturing of the formulation, wherein it is necessary to take into consideration that the active ingredient, such as, for example, budenoside, is not in contact with the ethanol until all the propellant has been added.

More specifically, the procedure to prepare the stable formulation of suspended budenoside of the present invention includes the following stages:
- Addition of the quantity of budenoside which is necessary to a thermostatted reactor, which is further provided with a stirring system.
- Addition of the hydrofluoralkane propellant (HFA-134a) to the thermostatted reactor.
- Activation of the reactor stirring system.
- Preparation of the ethanol solution of oleic acid, incorporating it in the reactor where the stirring is maintained.

Once the formulation has been prepared, each container is dosed with aerosol through the valve stem, by means, for example, of the Pamasol automatic machine. These containers have attached a suitable button for their oral inhalatory administration.

The dispensing valve used to dispense the formulation is manufactured in special materials, in particular those of the seal, which are compatible with the aerosol suspension, and which permit exactly dosing the medicament..

These valves are designed to release a measured quantity of the formulation by pressing and incorporate a seal to prevent the loss of propellant through the valve. The valve seal may be of any appropriate elastomeric material, such as, for example nitrile, a material which is very suitable for using with formulations which contain HFA134a and very small quantities of ethanol. These nitrile seals remain unaltered during the required time, making the aerosol correctly function dispensing exact quantities of medicament in each press of the aerosol.

The valve materials, especially the manufacturing material of the dosing chamber, is an inert material resistant to deformation of the contents of the formulation, in particular, ethanol. The materials particularly suitable for use in the manufacturing of the dosing chamber include polyesters, such as, for example, polybutylene terephthalate and acetals.

The formulation in accordance with the invention is packaged in aluminium metal receptacles, which may be coated or anodized, this container is closed with the dosing valve, which is capable of releasing a volume of between 25 mcl and 100 mcl, for example, 50 or 63 mcl, preferably, 50 mcl.

The stable formulations of suspended aerosols proposed by the invention, are especially suitable for the administration of medicaments through inhalatory route, and provide suspensions with a suitable resuspension time, so that they give precise doses and that the medicament does not adhere to the walls or floccule.

The stability of the formulation is controlled after its storage, by determination of the dosing uniformity of the valve, and the distribution of the aerodynamic size of the particles. Cascade impactors are used which permit determining that the respirable dose of medicament remains unaltered throughout the aging of the formulation.

The administration of the medicament to the patient is carried out through a button suitable for oral or nasal administration.

### EXAMPLES OF EMBODIMENT OF THE INVENTION

The examples described below illustrate the particular forms of embodiment of the present invention and does not aim in any way to limit the scope of protection thereof.

### Example 1: Aerosol formulation of budesonide (composition per 10 ml container):

An aerosol formulation of micronized budesonide was prepared by the combination of the active ingredient, dispersing agent, cosolvent and propellant which is indicated below:

| | |
|---|---|
| Micronized budesonide | 0.040 g |
| Absolute ethanol | 0.0307 g |
| Oleic acid | 0.0025 g |
| Propellant 134a | 12.2132 g |
| | 12.2864 g |

In each press of the inhaler applicator, 200 mcg of budesonide is released.

### Example 2: Aerosol formulation of budesonide (composition per 10 ml container):

An aerosol formulation of micronized budesonide was prepared by the combination of the active ingredient, dispersing agent, cosolvent and propellant which is indicated below:

| | |
|---|---|
| Micronized budesonide | 0.01 g |
| Absolute ethanol | 0.0307 g |
| Oleic acid | 0.0025 g |
| Propellant 134a | 12.2132 g |
| | 12.2564 g |

In each press of the inhaler applicator, 50 mcg of budesonide is released.

### Example 3: Aerosol formulation of fluticasone (composition per 6 ml container):

An aerosol formulation of fluticasone propionate was prepared by the combination of the active ingredient, dispersing agent, cosolvent and propellant which is indicated below:

| | |
|---|---|
| Fluticasone propionate | 0.030 g |
| Absolute ethanol | 0.0184 g |
| Oleic acid | 0.0015 g |
| Propellant 134a | 7.3279 g |
| | 7.3778 g |

In each press of the inhaler applicator, 250 mcg of fluticasone is released.

### Example 4: Aerosol formulation of fluticasone (composition per 6 ml container):

The same formulation as example 2 was prepared, but adding 0.015 g of propylene glycol per container, the composition thus being:

| | |
|---|---|
| Fluticasone propionate | 0.030 g |
| Absolute ethanol | 0.0184 g |
| Oleic acid | 0.0015 g |
| Propylene glycol | 0.0015 g |
| Propellant 134a | 7.3279 g |
| | 7.3793 g |

In each press of the inhaler applicator, 250 mcg of fluticasone is released.

### Example 5: Aerosol formulation of salmeterol (composition per 6 ml container):

An aerosol formulation of micronized salmeterol xinafoate was prepared by the combination of the active ingredient, dispersing agent, cosolvent and propellant which is indicated below:

| | |
|---|---|
| Salmeterol xinafoate | 0.0435 g |
| Absolute ethanol | 0.0184 g |
| Oleic acid | 0.0015 g |
| Propellant 134a | 7.3279 g |
| | 7.35215 g |

Each press releases 25 mcg of salmeterol.

### Example 6: Aerosol formulation of salmeterol (composition per 6 ml container):

A similar composition was prepared to that of example 4, but with a lower content in absolute ethanol:

| | |
|---|---|
| Salmeterol xinafoate | 0.0435 g |
| Absolute ethanol | 0.008 g |
| Oleic acid | 0.0015 g |
| Propellant 134a | 7.3437 g |
| | 7.35755 g |

Each press releases 25 mcg of salmeterol.

### Example 7:

Combinations of two active ingredients were also made (a bronchodilator and a corticosteroid), for example salmeterol xinafoate plus fluticasone propionate:

| | |
|---|---|
| Salmeterol xinafoate | 0.0435 g |
| Fluticasone propionate | 0.030 g |
| Absolute ethanol | 0.00184 g |
| Oleic acid | 0.0015 g |
| Propellant 134a | 7.3279 g |
| | 7.38215 g |

Each press releases 250 mcg of fluticasone and 25 mg of salmeterol.

### Example 8:

The same formulation was prepared as in example 6, but adding 0.0015 g of propylene glycol per container, the composition being as follows:

| | |
|---|---|
| Salmeterol xinafoate | 0.0435 g |
| Fluticasone propionate | 0.030 g |
| Absolute ethanol | 0.00184 g |
| Oleic acid | 0.0015 g |
| Propylene glycol | 0.0015 g |
| Propellant 134a | 7.3279 g |
| | 7.38365 g |

Each press releases 250 mcg of fluticasone and 25 mg of salmeterol.

All these formulations are filled in aluminium containers and are closed with dispensing valves of 50 mcl of chamber volume, from Valois, and whose seals are manufactured in nitrile material, said material has demonstrated great compatibility with the formulation, making the stability optimum and the product does not suffer any alterations during the shelf-life of the preparation.

## Claims

1. Novel stable formulation of suspended aerosols, especially devised for its application in the administration of medicaments by inhalatory route, **characterized in that** said formulation comprises one or more active ingredients, ethanol in a quantity less than 0.030 % w/w, a dispersing agent and a hydrofluorocarbonated propellant.

2. Novel stable formulation of suspended aerosols according to claim 1, **characterized in that** the active ingredients can be bronchodilators, in particular anticholinergics and sympathomimetics, such as for example ipatropium, triotropium, salbutamol, salmeterol, formoterol, terbutalin, isoproterenol, reproterol, bambuterol and pirputerol and anti-inflammatory corticosteroids, including beclomethasone, budesonide, flunisolide, fluticasone and triamcinolone.

3. Novel stable formulation of suspended aerosols according to claim 1, **characterized in that** the dispersing agent is selected from sorbitan trioleate, oleic acid or propylene glycol.

4. Novel stable formulation of suspended aerosols according to claim 3, **characterized in that** the dispersing agent is oleic acid, and it is used in a proportion lower than 0.050 % w/w, preferably 0.020 % w/w.

5. Novel stable formulation of suspended aerosols according to claim 1, **characterized in that** the hydrofluorocarbonated propellant is selected from the group formed by 1,1,1,2-tetrafluoroethane (134a), 1,1,1,2,3,3,3-heptafluoroethane (227) and their mixtures.

6. Novel stable formulation of suspended aerosols according to preceding claims, **characterized in that** the active ingredient is budesonide, the dispersing agent is oleic acid and the hydrofluorocarbonated propellant is 1,1,1,2-tetrafluoroethane (134a).

7. Novel stable formulation of suspended aerosols according to preceding claims, **characterized in that** the active ingredients are salmeterol and fluticasone, the dispersing agents are oleic acid and propylene glycol and the propellant is 1,1,1,2-tetrafluoroethane (134a).

8. Process to produce the stable formulation of suspended aerosols of the preceding claims, **characterized in that** it comprises:
- Addition of the quantity of budenoside which is necessary to a thermostatted reactor, which is further provided with a stirring system.
- Addition of the hydrofluoralkane propellant (HFA-134a) to the thermostatted reactor.
- Activation of the reactor stirring.
- Preparation of the ethanol solution of oleic acid, incorporating it in the reactor where the stirring is maintained.
